# EUROPEAN PATENT APPLICATION

(11) **EP 3 888 763 A1**
(43) Date of publication of application: **06.10.2021**
(21) Application number: 19888340.7
(22) Date of filing: 21.11.2019
(51) Int. Cl.: A63B 71/06, A63B 23/04, A63B 69/00, A63K 1/00, A63K 3/00

(54) **PACE-SETTING SYSTEM, PACE-SETTING PROGRAM, AND EXERCISE ASSISTANCE METHOD**

(30) Priority: 29.11.2018 JP 2018223045
(71) Applicant: Leomo, Inc., Boulder, Colorado 80301 (US)
(72) Inventor: KAJI, Kunihiko, Tokyo 141-0031 (JP)
(74) Representative: Grünecker Patent- und Rechtsanwälte PartG mbB
(86) International application number: PCT/JP2019/045597
(87) International publication number: WO 2020/110894

(57) **Abstract**

[Problem] To provide a pace setting system that can be constructed using only simple equipment at a low price, that has a high degree of freedom in terms of the installation location and applicable competition, and that can be used casually.

[Solution] A pace setting system that presents, to an athlete 2, pace information pertaining to speed, wherein the system comprises: a plurality of smartphones 1a-1n having a communication function and an information output function; and a coordination control unit 11c that controls, via the communication function of each of the plurality of information processing terminals, the timing at which information is outputted from the information output function of each of the information processing terminals. The coordination control unit 11c, which can accommodate a plurality of athletes, outputs pace information that corresponds to a speed setting for each of the athletes, the pace information being outputted at a timing corresponding to each moving body.

## Description

### FIELD OF THE INVENTION

The present invention relates to a pace setting system, a pace setting program, and an exercise assistance method for presenting information about the speed of a moving body such as a walking or running athlete to the moving body.

### BACKGROUND ART

In the past, for sports training such as walking or running, a variety of pace setting systems have been developed for keeping the pace of the athlete (for example, Patent Document 1). The pace setting system for competition disclosed in this Patent Document 1 is provided with the plurality of LED illumination elements arranged at regular intervals along the inner edge of the track of an athletics stadium, and controls the lighting of the LED illumination elements. This pace setting system for competition controls the LED illumination elements to successively turn on in accordance with a pace which is set up in advance so that a runner can run with reference to the LED illumination elements successively turning on (the speed of successively turning on the LED illumination elements).

### PRIOR ART DOCUMENTS

### PATENT DOCUMENT

Patent Document 1: Japanese Patent Published Application No. 2001-216546

### SUMMARY OF INVENTION

### PROBLEMS TO BE SOLVED BY THE INVENTION

However, in the case of the system disclosed in Patent Document 1, the installation becomes large with a number of LED illumination elements arranged on the track of an athletics stadium and the like so that the place of installation is limited, and the applicable competitions are also limited. In addition, there is a problem that a large amount of cost is needed for installing necessary equipment whose management and operating costs also becomes large.

In order to solve the problem as described above, it is an object of the present invention to provide a pace setting system, a pace setting program, and an exercise assistance method which can be implemented only with simple equipment at a low cost with a high degree of freedom in terms of the installation place and applicable competitions so that the system can be used without difficulty.

### MEANS TO SOLVE THE PROBLEMS

In order to accomplish the object as described above, the present invention is related to a pace setting system which presents information about a speed to a moving body, comprising:
a plurality of information processing terminals provided with a communication function and an information outputting function;
a cooperation control unit which controls timing of outputting information from the information outputting function of each information processing terminal through the communication function of each information processing terminal.

Also, the present invention is related to an exercise assistance method for presenting information about a speed to a moving body by the use of a plurality of information processing terminals provided with a communication function and an information outputting function, including a cooperation control step of controlling timing of outputting information from the information outputting function of each information processing terminal by a cooperation control unit through the communication function of each information processing terminal provided with the communication function and the information outputting function.

In the invention as described above, it is preferred that there are a plurality of moving bodies each of which is the aforementioned moving body, and that the cooperation control unit outputs information about a set speed of each of the plurality of the moving bodies with the timing corresponding to the each moving body. Also, in the invention as described above, it is preferred to further provide an installation information acquisition unit which acquires information about a position in which each information processing terminal is installed, and a presentation information setting unit which sets the information about the speed to be presented to the moving body, wherein the cooperation control unit controls the timing on the basis of the information given by the presentation information setting unit and the installation information acquisition unit.

Furthermore, in the invention as described above, it is preferred to further provide an imaging unit which is provided for each information processing terminal; an imaging control unit which performs an imaging operation with the imaging unit of each information processing terminal on the basis of the timing of outputting information by the cooperation control unit; and an editing unit which edits a video or still image taken by the imaging unit in accordance with a control history of the cooperation control unit.

Still further, in the invention as described above, it is preferred to further provide a position measurement unit which measures positional information of each information processing terminal, wherein the cooperation control unit controls the timing on the basis of the measurement result measured by the position measurement unit.

Incidentally, the system and the method in accordance with the present invention as described above can be implemented in a computer by running the program of the present invention described in a predetermined language. Namely, the program in accordance with the present invention is a pace setting program for presenting information about a speed to a moving body by the use of a plurality of information processing terminals provided with a communication function and an information outputting function, causing the information processing terminal to function as: a cooperation control unit which controls timing of outputting the information about the speed from the information outputting function of each information processing terminal through the communication function of each information processing terminal.

The system having the functionality as described above can be built to implement the method of the present invention by installing the program of the present invention in an IC chip or a memory device of a mobile terminal device, a smartphone, a wearable terminal, a mobile PC, another type information processing terminal, or a general purpose computer such as a personal computer or a server computer, and running the program on the CPU.

Namely, the program of the present invention can be distributed, for example, through a communication line, or as a package application which can be run on a stand-alone computer by storing the program in a computer readable storage medium. Such a storage medium includes a magnetic recording medium such as a flexible disk or a cassette tape, an optical disc such as CD-ROM or DVD-ROM, a RAM card and a variety of storage mediums. In addition, in accordance with the computer readable medium in which this program is stored, the above system and method can be easily implemented with a general purpose computer or a dedicated computer, and the program can be easily maintained, transported and installed.

### EFFECT OF THE INVENTION

As has been discussed above, in accordance with the present invention, since a pace setting system can be implemented by the use of simple equipment such as smartphones, the pace setting system can be built at a low cost with a high degree of freedom in terms of the installation place and applicable competitions so that the system can be used without difficulty.

### BRIEF DESCRIPTION OF DRAWINGS

[Fig. 1] Fig. 1 is a conceptual view showing the overall configuration of the pace setting system in accordance with an embodiment.
[Fig. 2] Fig. 2 is an explanatory view showing installation of the pace setting system in accordance with the embodiment.
[Fig. 3] Fig. 3 is a block diagram for showing the internal configuration of a communication terminal constituting the pace setting system in accordance with the embodiment.
[Fig. 4] Fig. 4 is a block diagram for showing the internal configuration of a management server constituting the pace setting system in accordance with the embodiment.
[Fig. 5] Fig. 5 is a flow chart for showing the overall operation of the pace setting system in accordance with the embodiment.
[Fig. 6] Fig. 6 is a flow chart for showing the operation of the pace setting system when installation information is generated in accordance with the embodiment.
[Fig. 7] Fig. 7 is a flow chart for showing the operation of the pace setting system when installation information is presented in accordance with the embodiment.
[Fig. 8] Fig. 8 is an explanatory view for showing exercise assistance (with a single moving body) by the pace setting system in accordance with the embodiment.
[Fig. 9] Fig. 9 is an explanatory view for showing exercise assistance (with a plurality of moving bodies) by the pace setting system in accordance with the embodiment.
[Fig. 10] Fig. 10 is an explanatory view for showing a cooperative imaging function of the pace setting system in accordance with the embodiment.
[Fig. 11] Fig. 11 is an explanatory view for showing operation of the pace setting system to select a course in accordance with the embodiment.
[Fig. 12] Fig. 12 is a flow chart for showing operation of the pace setting system in accordance with a modification example.
[Fig. 13] Fig. 13 is a flow chart for showing the operation of the pace setting system when installation information is presented in accordance with the modification example.

### BEST MODE FOR CARRYING OUT THE INVENTION

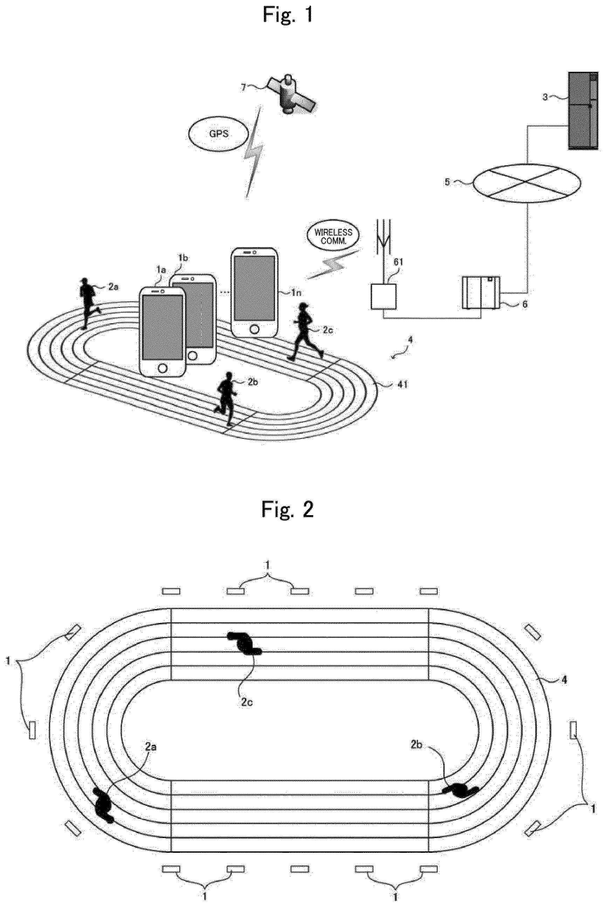

In what follows, a first embodiment of a pace setting system in accordance with the present invention will be explained with reference to the accompanying drawings. Fig. 1 is a conceptual view showing the overall configuration of the pace setting system in accordance with the present embodiment, and Fig. 2 is an explanatory view showing installation of the pace setting system in accordance with the present embodiment. Incidentally, the embodiment described below has been disclosed with devices and the like by way of illustration for implementing the technical idea of the present invention, which is not limited to the material, formation, structure, arrangement of each constituent member as described below. The technical idea of the present invention can be modified within the scope of claims.

### (Overall Configuration of Pace Setting System)

As shown in Fig. 1, the pace setting system in accordance with the present embodiment is a pace setting system for managing the pace of each athlete during running or walking or the like by making use of a plurality of smartphones 1, 1 as a pace setting system capable of performing data communication through a communication network 5 including wireless communication. More specifically, the pace setting system in accordance with the present embodiment includes, on the communication network 5, an application server 3, a relay device 6, and the plurality of smartphones 1, 1 possessed by each athlete 2, an administrative organization of an athletics stadium, a sport association and the like.

The communication network 5 including wireless communication is a distributed communication network which is constructed by connecting a variety of communication lines (public lines such as a telephone line, an ISDN line, an ADSL line and an optical line, a dedicated communication line, and a wireless communication network) to each other by the use of a variety of communication protocols such as TCP/IP. This communication network 5 may be a LAN such as an intranet (a network within a company), a home network, or the like based on 10BASE-T, 100BASE-TX or the like.

The smartphone 1, 1 is a portable information processing terminal provided with a communication function and a CPU and capable of performing a variety of functions by installing a variety of application software. In the case of the present embodiment, the smartphone can be used as a terminal device for pace setting system by installing a pace setting application. The information processing terminal for pace setting system can be implemented with, besides a smartphone, for example a functionally specialized dedicated device such as a game machine, and includes a tablet PC, a mobile computer, and a cellular phone.

In the case of the pace setting system of the present embodiment, this smartphone 1 is used as a device which provides information about the pace (speed or displacement) of each athlete to the athletes 2 and includes a communication function, an information output function and an imaging function such as a video camera. Specifically, the smartphone 1, 1 can receive communication services such as telephone conversation, data communication and so forth while the user is moving by wirelessly communicating with a relay point such as a usual base station through wireless communication.

In addition, the smartphone 1 is provided with a function for performing wireless communication with a wireless base station 61, and an information processing function for running an application. The communication system of this cellular phone may, for example, be a 4G system, an LTE system, a 3G system, an FDMA system, a TDMA system, a CDMA system, a W-CDMA system and the like. The smartphones 1, 1 establish wireless communication connection by these communication systems to provide telephone conversation and data communication via the smartphones 1a to 1c. Furthermore, this smartphone 1 is implemented with various functions such as a digital camera function, an application software execution function, a GPS function and the like.

The athletics stadium 4 is a ground on which a track 41 for competition is set up. In the case of the present embodiment, the athlete 2 performs a competition by running around the track 41 for competition. Then, as illustrated in Fig. 2 in the case of the present embodiment, the plurality of smartphones 1, 1 are arranged at a constant interval along the outer or inner course of the track 41 for competition.

The wireless base station 61 is connected to the communication network 5 through the relay device 6 and establishes wireless communication connection with the smartphones 1, 1 to provide telephone conversation and data communication by the smartphone 1. Incidentally, the wireless base station 61 of the present embodiment serves as a positional information acquisition unit to manage in which cell each mobile terminal resides on the communication network 5. Specifically, the positional information acquisition unit identifies the position of the smartphone 1 in response to a positional information request transmitted from the smartphone 1. In the case where the residing cell is switched because of moving of the user or the like, this position registration request is transmitted from the smartphone 1 to the wireless base station in charge of managing the cell in which the smartphone 1 resides anew, and therefore the smartphone 1 can grasp the own position on the basis of the base station identification information assigned to the base station. Meanwhile, this wireless base station may be a short distance communication base station such as a wi-fi router, wi-fi access point or the like.

The relay device 6 is a node device such as a modem, terminal adapter, gateway apparatus and the like for connecting with the communication network 5, and serves to perform relay operation between the wireless base station 61 and the communication network 5.

The application server 3 is an application server installed on the communication network 5 to remotely perform application management, device management, user management, operation status management, setting management, data collection management and the like. By these functions, the application server 3 distributes a pace setting application to the smartphone 1, notifies the smartphone 1 of various settings and management, collects data from the smartphone 1 and so forth.

### (Internal Configuration of Each Device)

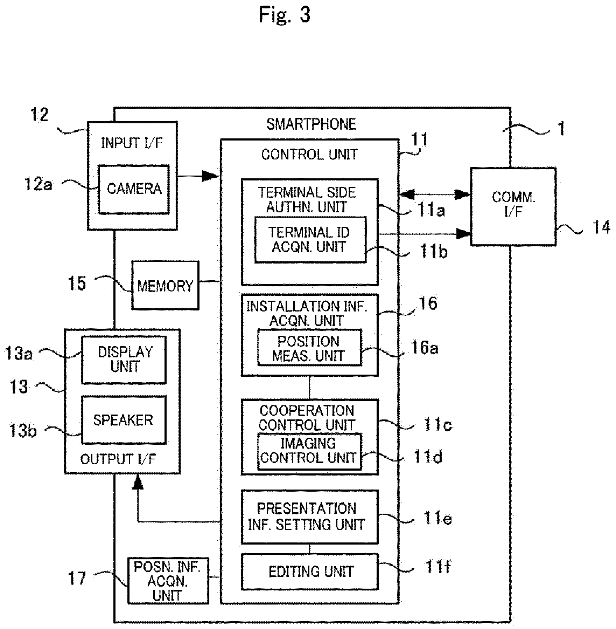

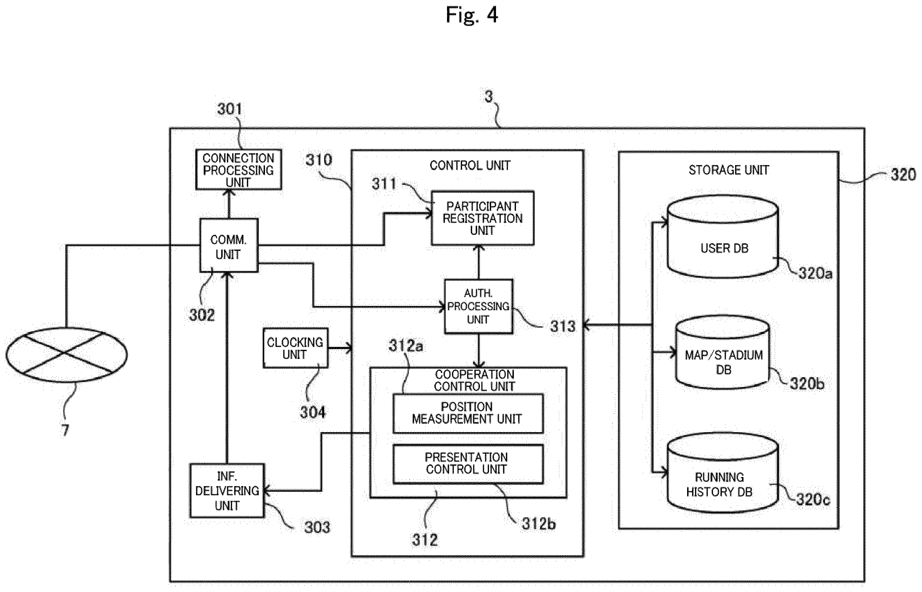

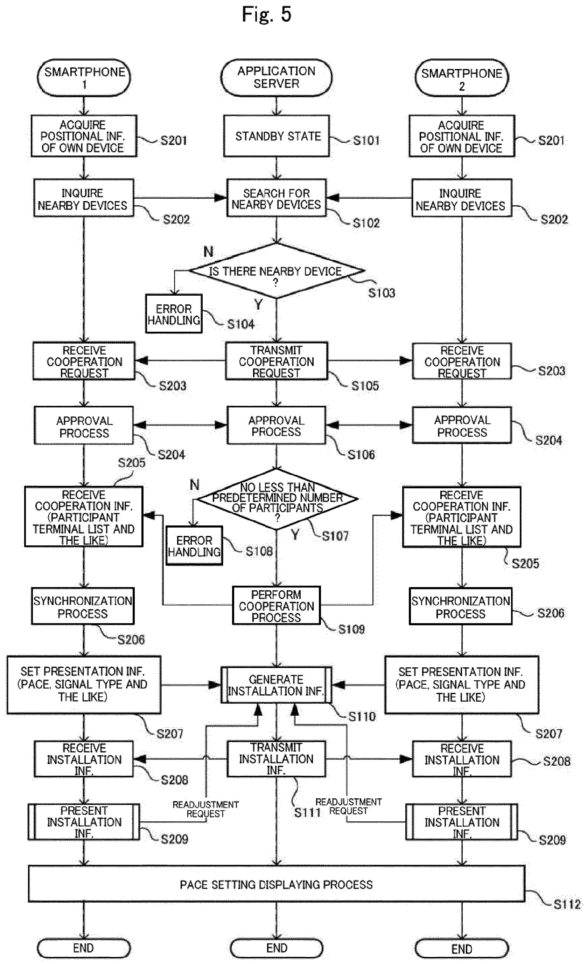

Next is an explanation of the internal configuration of each device constituting the pace setting system as described above. Fig. 3 is a block diagram for showing the internal configuration of the mobile terminal constituting the pace setting system in accordance with the present embodiment. Fig. 4 is a block diagram for showing the internal configuration of the wireless base station 61 constituting the pace setting system in accordance with the present embodiment. Fig. 4 is a block diagram for showing the internal configuration of the communication network 5 constituting the pace setting system in accordance with the present embodiment. Fig. 5 is a block diagram for showing the internal configuration of the application server 3 constituting the pace setting system in accordance with the present embodiment. Incidentally, the term "module" used in the following explanation stands for a function unit capable of performing a predetermined operation and implemented with hardware such as a device or apparatus, software capable of performing the functionality as required, or a combination thereof.

### (1) Smartphone 1

As illustrated in Fig. 3, the smartphone 1 is provided with an input interface 12, and an output interface 13 as user interface modules. The input interface 12 is a device such as an operation button, a touch panel and the like for inputting user operations, and includes a camera 12a as an imaging unit in the case of the present embodiment. On the other hand, the output interface 13 is a device such as a display 13a such as a liquid crystal display, a speaker 13b and the like for outputting images and sound. Particularly, the display unit 13a displays a GUI (Graphical User Interface) built by an application.

Furthermore, the smartphone 1 is provided with a positional information acquisition unit 17 The positional information acquisition unit 17 is a module for measuring the current position of own device and capable of calculating the coordinates of the current position on the basis of the GPS signal from a satellite 7, or acquiring the positional information of own device with reference to the base station identifiers of the wireless base stations 61 as communication partners of a communication interface 14 and the radio wave state such as signal intensities of the wireless base stations 61.

In addition to this, the smartphone 1 is provided with the communication interface 14 as a communication module. This communication interface 14 is provided with a communication function for performing telephone conversation and data communication through a general public line, and a short distance communication function on the basis of a protocol for data communication such as a wireless LAN. In the case of the present embodiment, the wireless base station 61 as an access point detects the smartphone 1 residing in the wireless communication range of own device and automatically sets up a communication link to form a hot spot of the wireless LAN and enable data communication through this communication interface 14. Also, in the case where a plurality of the smartphones 1 are linked to the access point, the smartphones 1, 1 can perform mutual data communication with each other.

Incidentally, the communication interface 14 is provided with a function to connect with the wireless base station 61 through wireless communication in collaboration with a terminal side authentication unit 11a and a terminal identifier acquisition unit 11b. The terminal side authentication unit 11a reads out an identifier (telephone number, IP address, and user ID) for identifying own device from the terminal identifier acquisition unit 11b, and transmits the identifier to the wireless base station 61 which is the connection partner to perform authentication. Meanwhile, when wireless LAN connection is made with the wireless base station 61, the process to establish connection may be started to form a link for performing data transmission and reception by monitoring radio signals from the outside and detecting an identifier for identifying the wireless base station 61.

Furthermore, the smartphone 1 is provided with a control unit 11 and a memory 15 as application execution modules. The control unit 11 is an arithmetic processing unit such as a CPU, and virtually builds function modules by running a variety of programs on this control unit 11. In the case of the present embodiment, a pace setting program is run on the control unit 11 to implement, as application execution modules, the terminal side authentication unit 11a, an installation information acquisition unit 16, a cooperation control unit 11c, a presentation information setting unit 11e and an editing unit 11f.

The terminal side authentication unit 11a is a module for performing part of the authentication process to participate in the pace setting system by performing user authentication for accessing the application server 3 on the network, detecting and mutually authenticating user terminals located in the vicinity (for example, terminals on the same wi-fi network). In the case of the present embodiment, the terminal side authentication unit 11a is provided with the terminal identifier acquisition unit 11b.

The terminal identifier acquisition unit 11b is a module for acquiring, when mutual authentication is performed between terminal devices, the device ID of the terminal device that is the subject of mutual authentication through the communication interface 14. Specifically, the positional information acquisition unit 17 exchanges the device IDs between terminals located in a certain area such as in the same communication cell, in a predetermined area on the basis of the latitude and longitude, in the same wi-fi network or the like, and mutually transmits and receives codes for authentication process to perform confirmation steps between terminals and form mutual links. By forming the mutual links, it is possible to participate in a group of terminals making up the same pace setting system and set up synchronization and cooperation among a plurality of terminals.

The cooperation control unit 11c is a module for performing a pace setting displaying process in cooperation with the application server 3 and other smartphones 1 participating in the group through the communication functionality thereof in order to control the content of pace information to be output from the output interface 13 of each smartphone 1 in cooperation with the application server 3 and a plurality of information processing terminals through the communication functionality thereof. In the case of the present embodiment, the cooperation with other smartphones 1 is performed through the application server 3. However, the information processing terminals can cooperate directly with each other. In this example, the smartphones 1 cooperate through the application server 3.

The cooperation control unit 11c outputs, through the output interface 13, the ideal moving speed, displacement or the like on the basis of the elapsed time of each athlete as pace information, for example, by having the display of the smartphone 1 emit specific color light with an increased brightness, or displaying the elapsed time or lap time of a stopwatch with enlarged characters.

This cooperation control unit 11c can output pace information of a single athlete, and pace information of multiple athletes at the same time. In this case, while a plurality of athletes participate in a running competition and a plurality of smartphones 1, 1 are arranged at a constant interval along the outer or inner course of the track 41 for competition as illustrated in Fig. 2, the plurality of smartphones 1, 1 successively display or emit light in accordance with pace information of each smartphone to output the pace information about the moving speed and displacement of each athlete. Namely, in the case where there are a plurality of athletes, the speed corresponding to the pace of each athlete (moving body) can be set up so that the cooperation control unit 11c outputs information about the set speed of each of the plurality of moving bodies with the timing corresponding to each moving body.

Furthermore, the cooperation control unit 11c of the present embodiment is provided with an imaging control unit 11d. The imaging control unit 11d is a module for performing imaging by the camera 12a of each information processing terminal on the basis of the information output timing of the cooperation control unit 11c. More specifically, in the case of the present embodiment, a video or still image is successively taken by the camera of each information processing terminal in synchronization with the pace of each athlete. While each athlete is running in accordance with own pace, the athlete passes by the front of the terminal at the moment when the terminal outputs pace information as long as the athlete is running at the pace. Because of this, the imaging control unit 11d can take a video or still image with the timing when outputting the pace information of each athlete or within a certain time width before and after the timing, and record the video or still image for each specific athlete. The video or still image as taken in this manner is processed by the editing unit 11f, for example, to perform concatenation for each athlete and create a video and a data file for each athlete.

The installation information acquisition unit 16 is a module for acquiring or setting the installation information of each information processing terminal to be installed, and accepts information necessary for calculating which terminal (the smartphone 1, 1) is installed in which position on the course such as a competition track for performing a competition to calculate installation positions on the basis of the information as accepted, and receive instructions from the application server 3 in operative association with the application server 3. The installation information acquisition unit 16 in accordance with the present embodiment is provided with a position measurement unit 16a.

The position measurement unit 16a is a module for measuring the positional information of each information processing terminal. The installation information acquisition unit 16 sets or acquires positional information by the use of the positional information measured by the position measurement unit 16a. More specifically, the installation information acquisition unit 16 calculates installation information on the basis of the current location of own device measured by the position measurement unit 16a and the positional information of other terminals, generates pace information to be displayed by querying the application server 3, and sets or acquires necessary information by receiving from the application server 3. The cooperation control unit 11c controls the timing of outputting a pace sign on the basis of this pace information.

The presentation information setting unit 11e is a module for setting information about the speed to be shown to a moving body. The cooperation control unit 11c controls the timing on the basis of settings given by the presentation information setting unit 11e and the installation information acquisition unit 16. More specifically, the presentation information setting unit 11e sends a request to a user for settings necessary to display pace information about the speed to be presented to each athlete as a moving body, and acquires identification information such as name, ID, nickname, account and the like for specifying each athlete, the pace which each athlete desires, and the values of sign types (illumination color, figure, character string and the like) to display the pace, and the acquired information is stored in a memory, notified to the application server 3 and so forth.

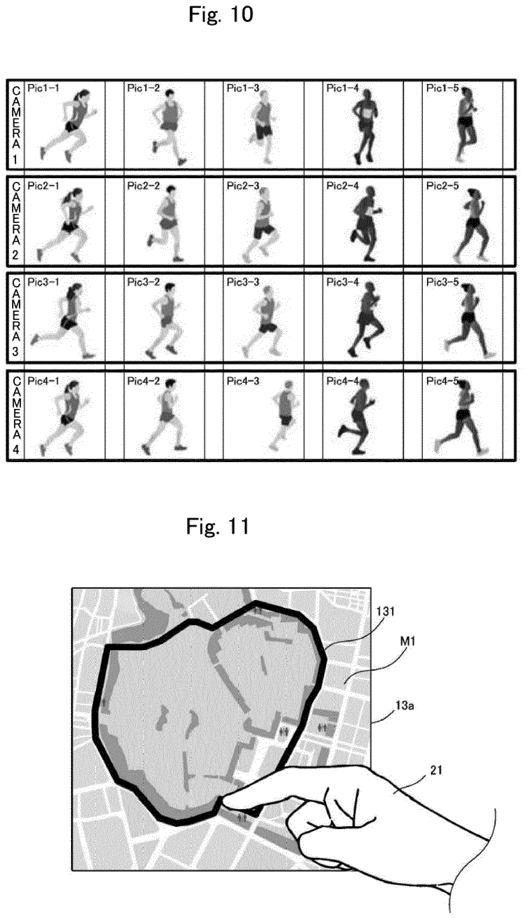

The editing unit 11f is a module for editing a video or still image taken by the camera 12a in accordance with the control history by the cooperation control unit 11c. More specifically, for example in the case where five athletes participate and are running at different paces as illustrated in Fig. 10, the cameras 1 to 4 of the smartphones take video or still images in accordance with the paces of the athletes respectively, and the editing unit 11f acquires/analyzes the control history and creates one video or a playlist by collecting and concatenating still images for each athlete such as Pic1-1 to 4-1, Pic1-2 to 4-2, Pic1-3 to 4-3, Pic1-4 to 4-4 and Pic1-5 to 4-5.

### (2) Application Server 3

As illustrated in Fig. 4, the application server 3 is provided with as communication modules, a communication unit 302, a connection processing unit 301 and a working information notification unit 303, and a control unit 310 as a user management module. Furthermore, this control unit 310 is connected to a storage unit 320 for recording various data and a clocking unit 304 for acquiring the current time.

The communication unit 302 is a communication device for transmitting and receiving various data to/from the communication network 5 by transmitting and receiving packet data. In the case of the present embodiment, this communication unit 302 is provided with a Web server function for delivering Web pages on the Internet and an email receiving function, and performs accepting access to a Web site from the smartphones 1 participating in the pace setting system and transmitting and receiving data to/from the smartphones 1 by protocols such as HTTP, SMTP or the like through which it is possible to acquire or set information necessary for building the pace setting system. The connection processing unit 301 is a module for establishing wireless communication with each smartphone 1 through the communication network 5.

The storage unit 320 serves as a database for accumulating information about the pace setting system. In the case of the present embodiment, the database includes a user DB 320a for storing information about users, a map/stadium DB 320b for storing information about a map of the whole country and athletic facilities, and an execution history DB 320c for storing information about the running history of the pace setting system.

The user DB 320a is a database device in which are registered users (athletes) who can use a pace setting application which is a computer program of the present invention, and implemented with a memory device in which are stored name, residence, telephone number, mail address, athletics event, past setting content/usage history and the like in association with user identification number (user ID). The map/stadium DB 320b is a database device in which are stored map information and facility information describing a map of the whole country, the coordinate positions, profiles, types and the like of athletics stadiums of the whole country. The execution history DB 320c is a database device for accumulating history information relating to running of the pace setting system to record various settings, the result of position measurement, the surrounding environment, actual running performance and the like when the pace setting system is running.

The clocking unit 304 is a module for acquiring the current time when needed. In the case of the present embodiment, the clocking unit 304 performs the process to transmit the current time when an authentication processing unit 313 performs an authentication process, and so forth.

The control unit 310 is an arithmetic operation module composed of hardware elements, for example, processor(s) such as a CPU and a DSP (Digital Signal Processor), a memory, and other necessary electronic circuits, software such as programs for implementing necessary functions of the hardware elements, or combination thereof. Several function modules can be virtually implemented by loading and executing the programs so that a variety of processes are performed by the implemented function modules in response to the operation by the user. In the case of the present embodiment, a participant registration unit 311, the authentication processing unit 313 and a cooperation control unit 312 are virtually implemented on the control unit 310 by running the pace setting program in accordance with the present invention.

The authentication processing unit 313 is a module for acquiring user identification information such as a user ID received from each smartphone 1, and performing an authentication process on the basis of this user identification information. More specifically, the authentication processing unit 313 is a computer or software capable of verifying the legitimacy of an accessing person, and confirms whether or not the accessing user has right qualification, whether or not the accessing user is the qualified person and so forth by acquiring a user ID through the communication network 5 to collate them with the user DB 320a. Then, the authentication processing unit 313 transmits the authentication result information to the cooperation control unit 312.

The participant registration unit 311 is a module for accepting user registration or provisional temporary registration required for receiving the pace setting service in accordance with this system, and recording the registration on the user DB 320a. In this case, when user registration is made, the participant registration unit 311 registers a user ID which is a user identification number as an identifier for specifying the user, and registers name, residence, mobile telephone number, mail address, athletics event, default pace information and the like as user information.

The cooperation control unit 312 is a module for receiving various inquiries and requests from each smartphone 1 and setting information provided by users, and performing arithmetic operations, data collection and data reception/transmission necessary for providing the pace setting service. Also, in the case of the present embodiment, the cooperation control unit 312 is provided with a position measurement unit 312a and a presentation control unit 312b.

The position measurement unit 312a is a module for measuring the current position of each smartphone 1. The current position of each smartphone 1 can be acquired by calculating the coordinates of the current position on the basis of GPS signals from the satellites 7, the base station identifier of the wireless base station 61 connected to the communication interface 14 of each terminal and the radio wave state such as signal intensities of the wireless base stations 61.

The presentation control unit 312b is a module for setting and generating information (pace information) about speeds to be shown to athletes as moving bodies, and assists or performs part or all functions of the cooperation control unit 11c and the presentation information setting unit 11e of each terminal to lessen processing burden on the user terminal and improve the processing speed.

More specifically, the presentation control unit 312b generates the installation information about the installation position of each terminal on the basis of the settings by the installation information acquisition unit 16 and the presentation information setting unit 11e of the terminal, and delivers the installation information to each terminal. Receiving this installation information of each terminal, the cooperation control unit 11c controls the output timing of pace information. Specifically, the presentation control unit 312b acquires the settings necessary for displaying pace information about the speed to be presented to each athlete through the presentation information setting unit 11e of each terminal, and manages the operation of the entirety of the system by collecting information about the installation position of each terminal to calculate and acquire the output timing of the pace information of each terminal.

### (Exercise Assistance Method)

The exercise assistance method in accordance with the present invention can be implemented by operating the pace setting system having the structure as described above. In the case of the present embodiment, an example will be explained in the case where each smartphone 1 accesses the application server 3, and cooperation control is executed in the application server 3 side. Incidentally, the procedure steps will be explained below simply as an example, and can be modified as much as possible. Also, with respect to the procedure steps as explained below, it is possible to omit, replace and add some steps readily in accordance with the embodiment.

### (1) Process For Pace Setting System Construction

At first, the process for constructing the pace setting system will be explained. Figs. 5-7 are flow charts for showing the operation of the pace setting system in accordance with the present embodiment. Incidentally, the application server 3 is always in a standby state and ready for accepting access from each smartphone.

In each smartphone side, a pace setting application is invoked which first acquires the positional information of own device (S201), and transmits a search request together with this positional information of own device to the application server 3 to inquire nearby devices (S202). Receiving this search request, the application server 3 performs searching for nearby devices (S202). In this case, the application server 3 communicates with the terminals that are running the pace setting application, and searches for the terminals located in the predetermined area by referring to the positional information contained in the search request from the terminal (S102).

When no nearby device is detected in the predetermined area, the application server 3 performs the process to have the requesting terminal displays error indication and so forth ("N" in S103). Conversely, when an nearby device(s) is detected in the predetermined area, the application server 3 transmits a cooperation request to the each detected device(s) (S105). Receiving this cooperation request (S203), the smartphone 1 prompts the user to perform operation for approval process (S204). In this approval process, the terminal returns whether to participate in the pace setting system on the basis of the user's operation.

Receiving this approval process by the user's operation, the application server 3 counts the number of terminals approving the cooperation request to determine whether or not a sufficient number of terminals are available to construct the pace setting system. If the number of terminals is no less than a predetermined number, it is confirmed that the pace setting system is established to start a cooperation process ("Y" in S107, S109). Conversely, if the number of terminals is less than the predetermined number in step S107, it is confirmed that the pace setting system cannot be established to perform an error handling, for example, by having the terminals display the message that the system cannot be established ("N" in S107, S108).

When no less than the predetermined number of terminals have been participated and it is determined that the pace setting system can be established ("Y" in S107), the cooperation process is performed between the application server 3 and each terminal (S109). In this cooperation process, cooperation information is transmitted to each terminal (S205). This cooperation information includes a participant terminal list containing the identifier, coordinates and network address for specifying each terminal participating in the system. Each terminal performs a synchronization process to establish communication connection for enabling mutual communication with the terminals listed in the participant terminal list by the use of this cooperation information (S206).

After completing this synchronization process, each terminal sets presentation information (S207). This presentation information is setting information about the speed to be shown to each athlete as a moving body and includes identification information such as name, ID, nickname, account and the like for specifying each athlete, the pace which each athlete desires, and the sign types (illumination color, figure, character string and the like) for displaying the pace. This presentation information can be set with various values by arbitrary operation performed by the user through the presentation information setting unit 11e. The presentation information which is set is transmitted to the installation information acquisition unit 16 or the application server 3 to generate installation information.

Receiving the presentation information, the application server 3 generates the installation information (S110) when all the presentation information is available. This installation information is information used to instruct which terminal is installed in which position on the competition course such as a competition track, generated by the application server 3 and delivered to each terminal (smartphone 1, 1) (S110, S111 and S208).

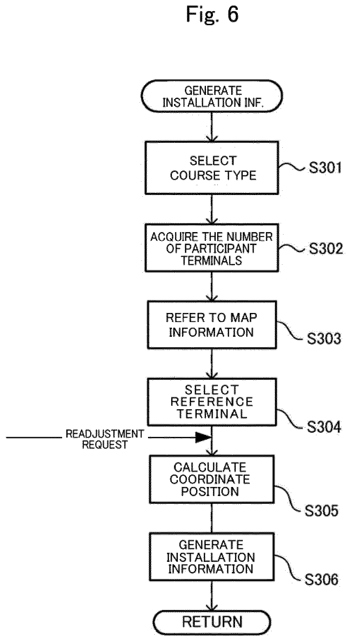

Fig. 6 is a flow chart for showing the procedure of an installation information generating process in step S110. When presentation information from all the terminals is received by the server side, an installation information generating procedure is started, and a course type is selected (S301). The course type can be selected by user input after inquiring of the terminal on the user side, or set up in advance by the user in step S207. Alternatively, the course type can be determined by specifying the location of the athletics stadium on the basis of the current position of the athlete 2 (smartphone 1, 1), and acquiring information about the type, total length, profile and the like of the athletics stadium with reference to a database. Furthermore, as illustrated in Fig. 11, the course type can be acquired as a profile by showing the map of the periphery of the current position of the athlete 2 in the smartphone 1, and having the user trace the running course on the map through a hand-writing input system.

In addition to this, the number of the terminals participating in building the pace setting system is acquired (S302). The acquisition of this number of the terminals is performed by counting the number of the data items of the received presentation information. Next, the profile, path and full length of the course selected in step S301 are read out by referring to the map information (S303). Meanwhile, in this case, if the map information and athletics stadium information have already been read out when selecting the course type in step S301, this step S303 can be dispensed with.

In the case of the present embodiment, a reference terminal is selected as a representative of the participating terminals (S304). This reference terminal is a terminal to be located in reference coordinates with which the installation coordinates of each terminal can be calculated, and may be the terminal that is the first to inquire in step S202, the terminal that is located closest to the start position of the course, the terminal that is arbitrarily selected by the user, or the terminal that is selected in a various way. Then, the coordinate position in which each terminal is arranged is calculated (S305) in accordance with the profile and total length of the course on the coordinate system with the selected reference terminal as the origin. This arrangement of the terminals is determined by dividing the total length of the course with the number of the terminals to calculate the interval between adjacent terminals and setting the coordinate position of each terminal in order that the terminals are arranged at uniform intervals.

However, in the case where it is difficult to install a terminal in step S305 because of terminals having been already installed or a geographical environmental problem, the terminal can transmit a readjustment request including the coordinate position of own device. When this readjustment request is transmitted, the coordinate position of the terminal having transmitted the readjustment request is preferentially determined followed by determining the coordinate positions of the other terminals such that the interval is not uniform. The list of coordinate positions of the terminals determined in this manner is generated as installation information (S306). The installation information generated in this manner is transmitted to each terminal (S111 and S208), and supplied to the presentation process of the installation information (S209).

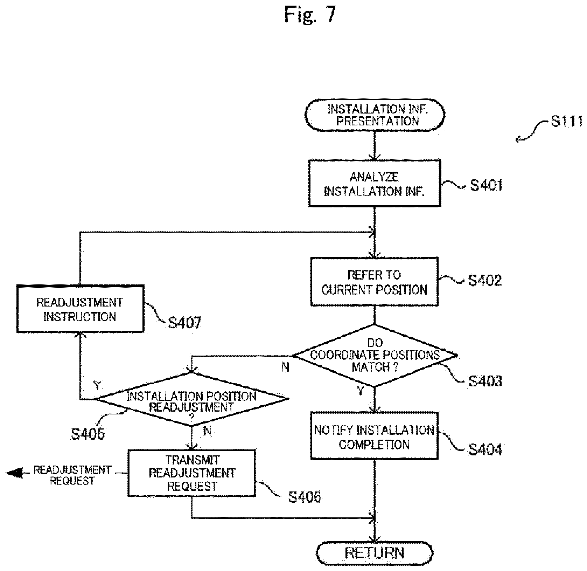

The installation information presentation process in step S209 is, as illustrated in Fig. 7, to analyze the installation information received by each terminal (S401), refer to the current position of own device (S402), and compare the current position of own device and the coordinate position indicated by the positional information to determine whether or not these coordinate positions match (S403). In the case where the current position of own device matches the coordinate position indicated by the installation information ("Y" in S403), the terminal notifies the user, the other terminals and the application server 3 that the installation is completed (S404). Conversely, in the case where the current position of own device does not match the coordinate position indicated by the installation information ("N" in S403), the terminal displays the displacement between the current position and the indicated coordinate position and inquires of the user whether to readjust the position.

The readjustment of the installation position is performed by moving the terminal itself to the coordinate position indicated by the installation information. If the readjustment of the installation position is possible or the user desires the readjustment ("Y" in S405), a readjustment instruction is displayed (S407) to prompt the user to move the terminal. Conversely, if the installation position of the terminal cannot be readjusted because of the environmental condition or the user does not desire the readjustment ("N" in S405), the terminal requires the server to readjust the installation information assigned to own device (S406). When this readjustment request is transmitted, the installation information presentation procedure is temporarily ended, and when installation information is transmitted anew from the application server 3, the installation information presentation procedure is performed again (S209).

The readjustment request transmitted to the server from this terminal is supplied to the installation information generating process in step S110 shown in Fig. 5, and installation information is generated by performing steps S305 and S306 again to calculate the coordinate position of the terminal to be readjusted, followed by transmitting the installation information again to the terminal having transmitted the readjustment request (S111 and S209).

Then, when the current position of own device match the coordinate position indicated by the positional information ("Y" in S403), the application server 3 and the other user terminals are notified of installation completion (S404). Thereafter, when installation completion is received from all the user terminals, a pace setting displaying process (S112) can be executed.

### (2) Process When Pace Setting System Is Operating

The process of the pace setting system implemented as has been discussed above will be explained in the case where information about speeds is shown to each athlete as exercise assistance. Basically, the output timing of pace signs (pace information) is controlled in accordance with the pace of each athlete on the basis of the presentation information and the installation information transmitted and received through the communication function of each smartphone 1, 1.

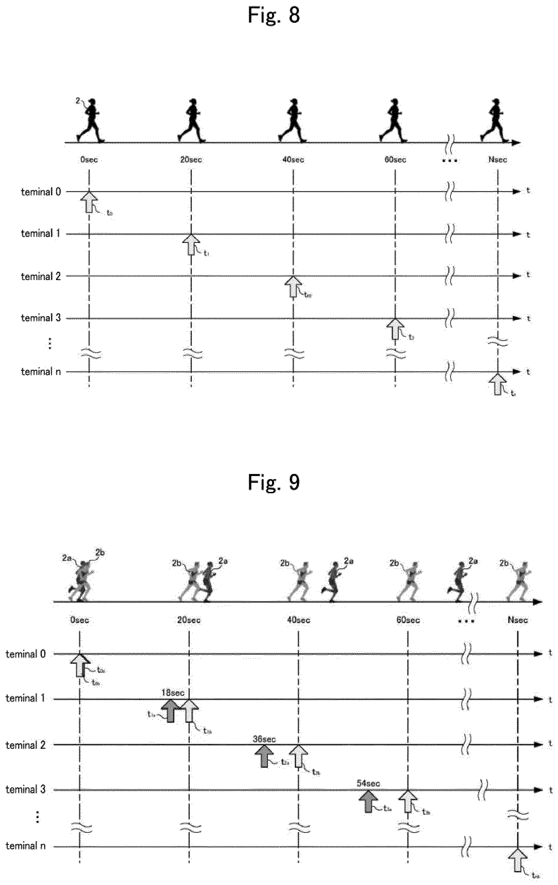

This pace information is successively issued in synchronization with the running speed of each athlete from the smartphone 1, 1 arranged at predetermined intervals along a competition course by outputting messages and sounds. For example, in the case where the system is set up in order that the athlete 2 wants to run at a pace of 18 kilometers an hour (5 meters a second) while the smartphones are arranged at intervals of 100 meters, the smartphones successively turn on so as to relay indication of a pace sign every 20 seconds as illustrated in Fig. 8. By this configuration, when the athlete 2 starts the start point at time t0, the smartphone 0 located at the start point outputs the pace sign. The display of the pace sign is successively relayed along the course as the next smartphone 1 outputs the pace sign after 20 seconds, i.e., time t1, and the further next smartphone 2 outputs the pace sign further after 20 seconds. The athlete 2 can keep his pace by running in concordance with the moving speed of the pace sign.

Also, in the case where there are athletes 2a and 2b running at different paces, for example in the case where the system is set up in order that the athlete 2b wants to run at a pace of 18 kilometers an hour (5 meters a second) and the athlete 2a wants to run at a pace of 20 kilometers an hour (5.5 meters a second) while the smartphones are arranged at intervals of 100 meters, the smartphones successively turn on so as to relay indication of a pace sign every 20 seconds for the athlete 2b and relay indication of a pace sign every 18 seconds for the athlete 2a as illustrated in Fig. 9. By this configuration, when the athletes 2a and 2b start the start point at time t0, the smartphone 0 located at the start point outputs the pace sign (in this case, the two athletes overlap so that only either sign is output).

Then, the displays of the pace signs are successively relayed at respective paces (speeds) along the course such as the pace sign for the athlete 2a is output at time t1a about 18 seconds after starting, and the pace sign for the athlete 2b is output 2 seconds thereafter, i.e., at time t1b about 20 seconds after starting. The athletes 2a and 2b can keep their pace by running in concordance with the moving speeds of the pace signs respectively.

### (3) Operation Of Imaging And Editing Process

Next is an explanation of the operation of taking a video or still image of the running form of each athlete, and editing the video or still image to create a sequence of photographs or a sequence of videos for each athlete. Fig. 10 is an explanatory view for showing the operation of an imaging and editing process.

Basically, each smartphone is provided with the camera 12a as an imaging means. An image of each athlete is taken at the timing when a pace information is output by the smartphone which outputs the pace information. Each video or still image is edited by the editing unit 11f in accordance with the control history of the cooperation control unit 11c after finishing the competition.

For example in the case where five athletes participate and are running at different paces as illustrated in Fig. 10, the smartphones performs output operation by emitting light or the like as a sign in synchronization with the pace of each athlete. The cameras 1 to 4 take video or still images in synchronization with the output timing. In the case of a video, the video is taken for several seconds before and after the timing of outputting a sign. This imaging operation is recorded as a control history. In the case of the example shown in the figure, the cameras 1 to 4 take images in synchronization with the paces of the athletes respectively as Pic1-1 to 1-5, Pic2-1 to 2-5, Pic3-1 to 3-5 and Pic4-1 to 4-5. Thereafter, the editing unit 11f acquires/analyzes the control history and creates one video or a playlist for each athlete by collecting and concatenating still images such as Pic1-1 to 4-1, Pic1-2 to 4-2, Pic1-3 to 4-3, Pic1-4 to 4-4 and Pic1-5 to 4-5.

### (Modification Example)

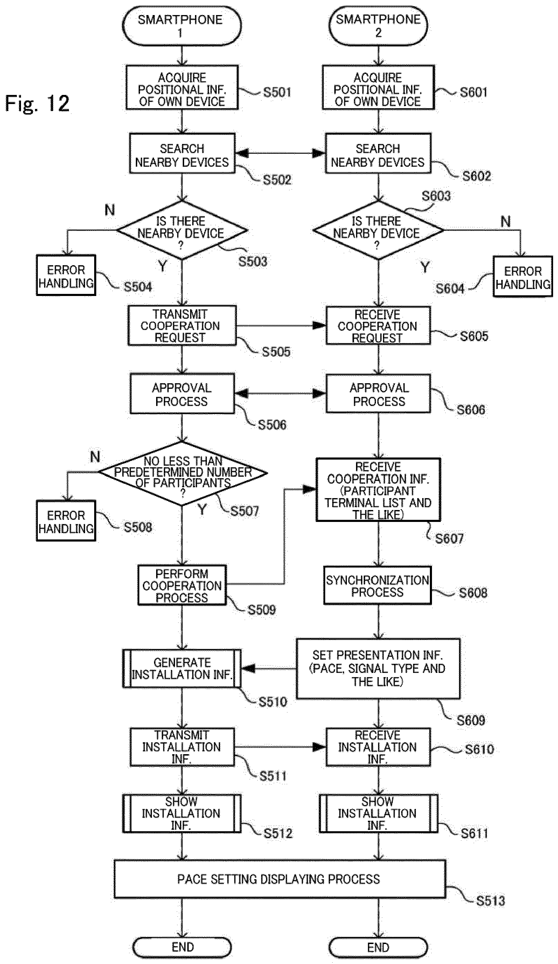

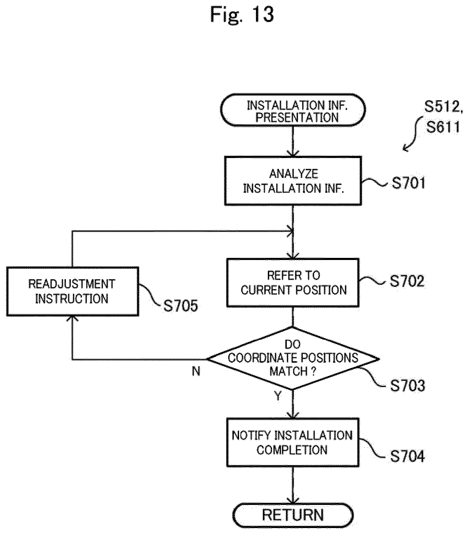

Incidentally, the above explained embodiment shows only one example of the present invention. Because of this, the present invention is not limited to the above embodiment, and various modifications are possible in accordance with the design and so forth without departing from the technical spirit of the invention. For example, while the application server 3 is installed to perform various processes and have the terminals cooperate with each other through the application server 3, the present invention is not limited thereto but the application server 3 can be dispensed with so that a pace setting system can be made up only with the terminals by coordinating information among the terminals. Specifically, as illustrated in Fig. 12 and Fig. 13, the smartphones 1, 1 as the user terminals are directly cooperating with each other to operate the system. Also in this case, the execution of the pace setting displaying process itself is similar to that of the embodiment as described above, but there are differences in the process and operation during the installation of each terminal. Here is an explanation of the process to build a pace setting system only with the user terminals.

In each smartphone 1, a pace setting application is invoked which first acquires the positional information of own device (S501,S601), and transmits a participation inquiry together with this positional information of own device to nearby terminals (S502, S602). The search for nearby terminals is continued for a predetermined period after a starting operation at each terminal to search for a terminal transmitting a participation inquiry in this period. For example, in this case, the terminals located in the predetermined area are detected by performing communication with nearby terminals which are running the pace setting application, i.e., the terminals in the same wi-fi network, and mutually referring to the positional information of the terminals transmitting participation inquiries.

In the case where there is no detected device located in the nearby area, the terminal performs the process to display error indication to the user and so forth ("N" in S504, S604). Conversely, when an nearby device(s) is detected in the predetermined area, a leading terminal transmits a cooperation request to the each detected device(s) (S505). With respect to this leading terminal, various methods can be employed to determine which terminal is to be this leading terminal. For example, the terminal which first starts search in the nearby area may be determined as the leading terminal in the following session. Alternatively, a specific terminal may be selected as the leading terminal in advance among users. Furthermore, for example, in the case where the participating terminals are within a known athletics stadium, the terminal located closest to the start point may be determined as the leading terminal.

Then, the smartphone 1b which receives a cooperation request (S605) from the leading terminal (the smartphone 1 in this case) requires the user to perform operation for approval process (S606). In this approval process, the smartphone returns whether to participate the pace setting system on the basis of the user's operation.

Receiving this approval process by the user's operation, the leading terminal 1a counts the number of terminals approving the cooperation request to determine whether or not a sufficient number of terminals are available to construct the pace setting system. If the number of terminals is no less than a predetermined number, it is confirmed that the pace setting system is established to start a cooperation process ("Y" in S507, S509). Conversely, if the number of terminals is less than the predetermined number in step S507, it is confirmed that the pace setting system cannot be established to perform an error handling, for example, by having each terminal display the message that the system cannot be established ("N" in S507, S508).

When no less than the predetermined number of terminals have been participated and it is determined that the pace setting system can be established ("Y" in S507), the cooperation process is performed between the leading terminal 1a and each terminal (S509). In this cooperation process, cooperation information is transmitted to each terminal (S607). This cooperation information includes a participant terminal list containing the identifier, coordinates and network address for specifying each terminal participating in the system. Each terminal performs a synchronization process to establish communication connection for enabling mutual communication with the terminals listed in the participant terminal list by the use of this cooperation information (S608).

After completing this synchronization process, each terminal sets presentation information (S609). This presentation information is setting information about the speed to be shown to each athlete as a moving body and includes identification information such as name, ID, nickname, account and the like for specifying each athlete, the pace which each athlete desires, and the sign types (illumination color, figure, character string and the like) for displaying the pace. This presentation information can be set with various values by arbitrary operation performed by the user through the presentation information setting unit 11e. The presentation information which is set is transmitted to the installation information acquisition unit 16 or the leading terminal 1a to generate installation information.

Receiving the presentation information, the leading terminal 1a generates the installation information (S510) when all the presentation information is available. This installation information is information used to instruct which terminal is installed in which position on the competition course such as a competition track, generated by the leading terminal 1a and delivered to the other terminals (smartphone 1, 1) (S511 and S610). The installation information generating process is the same as shown in Fig. 6. Namely, when presentation information from all the terminals is received by the leading terminal 1a side, an installation information generating procedure is started, and a course type is selected (S301).

The course type can be selected by user input after inquiring of the user through the leading terminal or other terminals, or set up in advance by the user in step S609. Alternatively, the course type can be determined by specifying the location of the athletics stadium on the basis of the current position of the athlete 2 (smartphone 1, 1), and acquiring information about the type, total length, profile and the like of the athletics stadium with reference to a database. Furthermore, as illustrated in Fig. 11, the course type can be acquired as a profile by showing the map of the periphery of the current position of the athlete 2 in the smartphone 1, and having the user trace the running course on the map through a hand-writing input system.

In addition to this, the number of the terminals participating in building the pace setting system is acquired (S302). The acquisition of this number of the terminals is performed by counting the number of the data items of the received presentation information. Next, the profile, path and full length of the course selected in step S301 are read out by referring to the map information (S303). Meanwhile, in this case, if the map information and athletics stadium information have already been read out when selecting the course type in step S301, this step S303 can be dispensed with.

In the case of the present embodiment, a reference terminal is selected as a representative of the participating terminals (S304). This reference terminal is a terminal to be located in reference coordinates with which the installation coordinates of each terminal can be calculated, and may be the terminal that is functioning as the leading terminal, the terminal that is located closest to the start position of the course, the terminal that is arbitrarily selected by the user, or the terminal that is selected in a various way. Then, the coordinate position in which each terminal is arranged is calculated (S305) in accordance with the profile and total length of the course on the coordinate system with the selected reference terminal as the origin. This arrangement of the terminals is determined by dividing the total length of the course with the number of the terminals to calculate the interval between adjacent terminals and setting the coordinate position of each terminal in order that the terminals are arranged at uniform intervals.

However, in the case where it is difficult to install a terminal in step S305 because of terminals having been already installed or a geographical environmental problem, the terminal can transmit a readjustment request including the coordinate position of own device. When this readjustment request is transmitted, the coordinate position of the terminal having transmitted the readjustment request is preferentially determined followed by determining the coordinate positions of the other terminals such that the interval is not uniform. The list of coordinate positions of the terminals determined in this manner is generated as installation information (S306). The installation information generated in this manner is transmitted to the other terminals (S511 and S610), and supplied to the presentation process of the installation information (S512 and S611).

In the case of this modification example, each terminal can perform the installation information presentation process in steps S512 and S611 independently on the basis of the own determination by the positional information acquisition function implemented within each terminal itself. More specifically, as illustrated in Fig. 13, the installation information presentation process is to analyze the installation information received by each terminal (S701), refer to the current position of own device (S702), and compare the current position of own device and the coordinate position indicated by the positional information to determine whether or not these coordinate positions match (S703). In the case where the current position of own device matches the coordinate position indicated by the installation information ("Y" in S703), the terminal notifies the user, the other terminals and the leading terminal 1a that the installation is completed (S704).

Conversely, in the case where the current position of own device does not match the coordinate position indicated by the installation information ("N" in S703), the terminal displays the displacement between the current position and the indicated coordinate position and inquires of the user whether to readjust the position. The readjustment of the installation position is performed by moving the terminal itself to the coordinate position indicated by the installation information.

Then, when the current position of own device match the coordinate position indicated by the installation information ("Y" in S703), the application server 3 and the other user terminals are notified of installation completion (S704). Thereafter, when installation completion is received from all the user terminals, a pace setting displaying process (S513) can be executed as illustrated in Fig. 12.

### (Effect/Action)

In accordance with the present embodiment as discussed above, since a pace setting system can be implemented by the use of simple equipment such as smartphones, the pace setting system can be built at a low cost with a high degree of freedom in terms of the installation place and applicable competitions so that the system can be used without difficulty. Incidentally, the present invention is not limited to the above described embodiments as they are, but it is possible to modify the constituent elements for embodiment without departing from the sprit of the present invention. Also, a variety of inventions may be embodied by appropriately combining a plurality of constituent elements disclosed in the above described embodiments. For example, it is possible to dispense with some elements of all the constituent elements disclosed in the embodiments.

### Explanation of Symbols

- 1: mobile terminal
- 2: athlete
- 3: application server
- 4: athletics stadium
- 5: communication network
- 6: relay device
- 7: satellite
- 11: control unit
- 11a: terminal side authentication unit
- 11b: terminal identifier acquisition unit
- 11c: cooperation control unit
- 11d: imaging control unit
- 11e: presentation information setting unit
- 11f: editing unit
- 12: input interface
- 12a: camera
- 13: output interface
- 13a: display unit
- 13b: speaker
- 14: communication interface
- 15: memory
- 16: installation information acquisition unit
- 301: connection processing unit
- 302: communication unit
- 303: working information notification unit
- 304: clocking unit
- 310: control unit
- 311: participant registration unit
- 312: cooperation control unit
- 312a: position measurement unit
- 312b: presentation information control unit
- 313: authentication processing unit
- 320: storage unit
- 320a: user DB
- 320b: map/stadium DB
- 320c: execution history DB

## Claims

1. A pace setting system which presents information about a speed to a moving body, comprising:
a plurality of information processing terminals provided with a communication function and an information outputting function; and
a cooperation control unit which controls timing of outputting information from the information outputting function of each information processing terminal through the communication function of each information processing terminal.

2. The pace setting system of claim 1 wherein
there are a plurality of moving bodies each of which is the aforementioned moving body, and wherein
the cooperation control unit outputs information about a set speed of each of the plurality of the moving bodies with the timing corresponding to the each moving body.

3. The pace setting system of claim 1 or 2 further comprising:
an installation information acquisition unit which acquires information about a position in which each information processing terminal is installed, and
a presentation information setting unit which sets the information about the speed to be presented to the moving body, wherein
the cooperation control unit controls the timing on the basis of the information given by the presentation information setting unit and the installation information acquisition unit.

4. The pace setting system of any one of claims 1 through 3 further comprising:
an imaging unit which is provided for each information processing terminal;
an imaging control unit which performs an imaging operation with the imaging unit of each information processing terminal on the basis of the timing of outputting information by the cooperation control unit; and
an editing unit which edits a video or still image taken by the imaging unit in accordance with a control history of the cooperation control unit.

5. The pace setting system of claim 1 further comprising:
a position measurement unit which measures positional information of each information processing terminal, wherein
the cooperation control unit controls the timing on the basis of the measurement result measured by the position measurement unit.

6. A pace setting program for presenting information about a speed to a moving body by the use of a plurality of information processing terminals provided with a communication function and an information outputting function, causing the information processing terminal to function as:
a cooperation control unit which controls timing of outputting the information about the speed from the information outputting function of each information processing terminal through the communication function of each information processing terminal.

7. The pace setting program of claim 6 wherein
there are a plurality of moving bodies each of which is the aforementioned moving body, and wherein
the cooperation control unit outputs information about a set speed of each of the plurality of the moving bodies with the timing corresponding to the each moving body.

8. The pace setting program of claim 6 or 7 further causing the information processing terminal to function as:
an installation information acquisition unit which acquires information about a position in which each information processing terminal is installed, and
a presentation information setting unit which sets the information about the speed to be presented to the moving body, wherein
the cooperation control unit controls the timing on the basis of the information given by the presentation information setting unit and the installation information acquisition unit.

9. The pace setting program of any one of claims 6 through 8 further causing the information processing terminal to function as:
an imaging control unit which performs an imaging operation with an imaging unit of each information processing terminal on the basis of the timing of outputting information by the cooperation control unit; and
an editing unit which edits a video or still image taken by the imaging unit in accordance with a control history of the cooperation control unit.

10. The pace setting program of claim 9 wherein
a position measurement unit is further provided which measures positional information of each information processing terminal, and wherein
the cooperation control unit controls the timing on the basis of the measurement result measured by the position measurement unit.

11. An exercise assistance method for presenting information about a speed to a moving body by the use of a plurality of information processing terminals provided with a communication function and an information outputting function, including
a cooperation control step of controlling timing of outputting information from the information outputting function of each information processing terminal by a cooperation control unit through the communication function of each information processing terminal provided with the communication function and the information outputting function.

12. The pace setting method of claim 11 wherein
there are a plurality of moving bodies each of which is the aforementioned moving body, and wherein
the cooperation control unit outputs information about a set speed of each of the plurality of the moving bodies with the timing corresponding to the each moving body.

13. The pace setting method of claim 11 or 12 further comprising:
an installation information acquisition step for acquiring information about a position in which each information processing terminal is installed by an installation information acquisition unit; and
a presentation information setting step for setting the information about the speed to be presented to the moving body by a presentation information setting unit, wherein
in the cooperation control step, the timing is controlled on the basis of the information given by the presentation information setting unit and the installation information acquisition unit.

14. The pace setting method of any one of claims 11 through 13 further comprising:
an imaging unit which is provided for each information processing terminal;
an imaging control step for performing an imaging operation by an imaging control unit with the imaging unit of each information processing terminal on the basis of the timing of outputting information by the cooperation control unit; and
an editing step for editing, by an editing unit, a video or still image taken by the imaging unit in accordance with a control history of the cooperation control unit.

15. The pace setting method of claim 11 further comprising:
a position measurement step for measuring positional information of each information processing terminal by position measurement unit, and wherein
in the cooperation control step, the timing is controlled on the basis of the measurement result measured by the position measurement unit.
